# EUROPEAN PATENT SPECIFICATION

(11) **EP 1 142 930 B1**
(45) Date of publication and mention of the grant of the patent: **21.02.2007**
(21) Application number: 01109854.8
(22) Date of filing: 11.10.1995
(51) Int. Cl.: C08G 77/20, C08G 77/38, C07F 7/08

(54) **Novel benzylidene malonate siloxane**
Benzalmalonatgruppen enthaltendes Organosiloxan
Siloxane à fonction benzalmalonate

(30) Priority: 14.10.1994 EP 94810601
(43) Date of publication of application: 10.10.2001
(62) Divisional of application: 95116022.5
(73) Proprietor: DSM IP Assets B.V., 6411 TE Heerlen (NL)
(72) Inventor: Bernasconi, Pierclaudio, 1217 Meyrin (CH); Gonzenbach, Hans Ulrich, 1202 Geneva (CH)
(74) Representative: Keller, Günter

(56) References cited:
- EP-A- 0 305 059
- EP-A- 0 383 655
- EP-A- 0 709 080
- WO-A-92/20690
- WO-A-93/10745
- FR-A- 2 642 967

## Description

The present invention relates to a novel benzylidene malonate siloxane having the formula 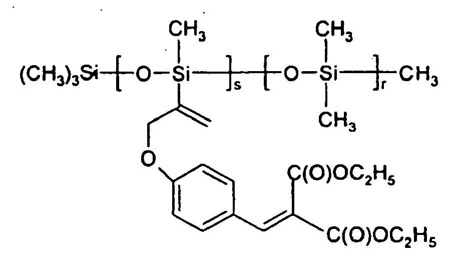 wherein s is ca. 4 and r is ca. 60, and which contains ca. 20 % of the alkylene isomer.

WO 92/20690 discloses benzylidene malonate siloxanes and a process for their preparation which generically includes the alkylene isomer of the above-identified compound, those alkylene isomers being characterized by the structural unit 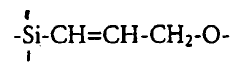 instead of the structural unit 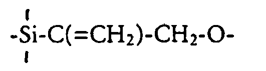 as shown in the above formula.

The novel benzylidene malonate siloxane is effective in absorbing ultra violet radiation in the erythemic region (290 - 320nm) which makes it particularly suitable for use in cosmetic sunscreen preparations where absorption in the UV-B region is particularly desirable. The novel benzylidene malonate siloxane can also be used in combination with dibenzoylmethane UV-A filters where it exerts a photostabilizing stabilizing effect on these filters as disclosed in EP 0 709 080. For example, 0,1 to 20 % by weight of the polymer filter is added to 0,5 to 5 % by weight of the dibenzoylmethane UV-A screening agent, the weight ratio of the silicone to the dibenzoylmethane derivative being not less than 0.1 and not more than 25.

The novel benzylidene malonate siloxane is lipophilic. The cosmetic formulations contain thus at least one fatty phase, and the formulations can consequently present themselves in the form of emulsions, lotions or gels.

Suitably the cosmetic screening composition takes the form of an oil, a lotion, a gel, a solid stick, an emulsion, e.g. cream, milk or of a vesicular dispersion of ionic or nonionic amphiphilic lipids, an aerosol, a spray, a foam, a powder, a shampoo, a hair conditioner or lacquer or a make-up, etc.

The usual solvents known to the skilled practitioner can be used for the preparation of these forms, e.g. oils, waxes, alcohols, polyols, etc. The preferred agents are fatty acids, esters, fatty alcohols, but also ethanol, isopropanol, propylene glycol, glycerine, etc.

The cosmetic formulations may contain further adjuvants, e.g. further solvents, thickeners, emollients, emulsifiers, humectants, tensides, preservatives, antifoams, fragrances, oils, waxes, lower polyols and monohydric alcohols, propellants, silicones, colourings and pigments, etc.

The preparation of the novel benzylidene malonate siloxane is illustrated below:

13.28 g of {[4-(2-propynyloxy)phenyl]methylene}-diethyl ester were dissolved in 75 g of toluene and heated under nitrogen to about 70°C. 44 g of a hydrosiloxane having a degree of polymerisation of 65 and 6 mpc SiH groups (2.36% SiH) were then added dropwise after a platinum complex was also added, giving 10⁻⁴ mole of Pt per mole of SiH of the hydrosiloxane. The mixture was heated to reflux and maintained until all SiH had disappeared of the infrared spectroscopic analysis. It was then allowed to cool to room temperature. The toluene was then evaporated to leave after washing 52 g of a brown, viscous polymer.

## Claims

1. **Benzylidene malonate siloxane of the formula** 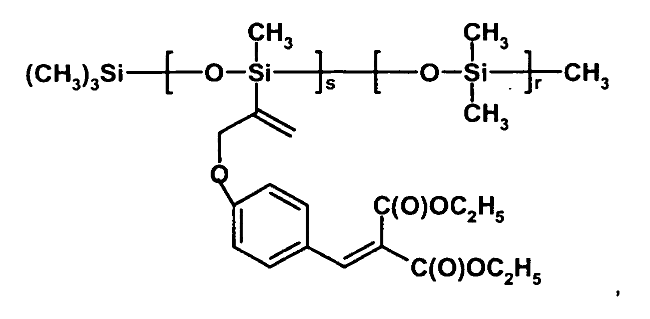 wherein s is ca. 4 and r is ca. 60, and which contains ca. 20% of the alkylene isomer.

2. Benzylidene malonate siloxane, obtainable by
a) dissolving 13.28 g of {[4-{2-propynyloxy)phenyl]methylene}-diethyl ester in 75 g of toluene and heating under nitrogen to about 70°C,
b) adding dropwise, after a platinum complex was also added, 44 g of a hydrosiloxane having a degree of polymerisation of 65 and 6 mpc SiH groups (2.36% SiH) to give 10⁻⁴ mole of Pt per mole of SiH of the hydrosiloxane,
c) heating the mixture to reflux and maintaining until all SiH had disappeared of the infrared spectroscopic analysis,
d) allowing the mixture to cool to room temperature, and
e) evaporating the toluene to leave after washing the brown, viscous polymer.

## Patentansprüche

1. Benzylidenmalonatsiloxan der Formel 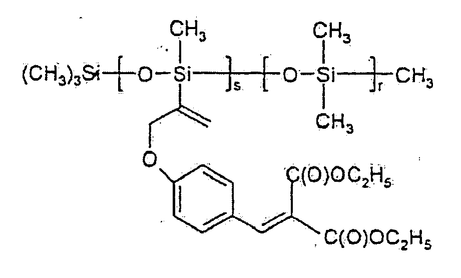 worin s ca. 4 ist und r ca. 60 ist und die ca. 20% des Alkylenisomers enthält.

2. Benzylidenmalonatsiloxan erhältlich, indem
a) 13,28 g {[4-(2-Propinyloxy)phenyl]methylen}diethylester in 75 g Toluol gelöst werden und unter Stickstoff auf etwa 70°C erhitzt werden,
b) nachdem ein Platinkomplex zugegeben worden ist, 44 g eines Hydrosiloxans tropfenweise zugegeben werden mit einem Polymerisationsgrad von 65 und mit 6 mpc SiH-Gruppen (2,36% SiH), sodass sich 10⁻⁴ Mol Pt pro Mol SiH des Hydrosiloxans ergeben,
c) die Mischung zum Rückfluss erhitzt wird und am Rückfluss gehalten wird, bis alles SiH gemäß Infrarotspektroskopieanalyse verschwunden ist,
d) die Mischung auf Raumtemperatur abkühlen gelassen wird und
e) das Toluol verdampft wird, was nach dem Waschen das braune viskose Polymer zurücklässt.

## Revendications

1. Siloxane à fonction benzalmalonate de formule 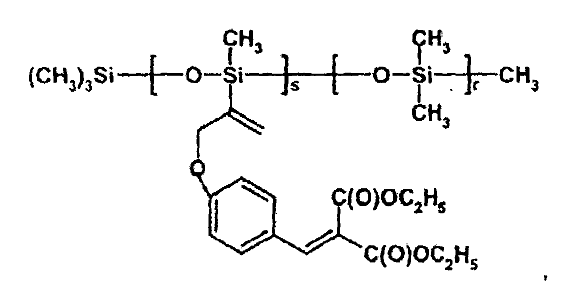 dans laquelle s vaut environ 4 et r vaut environ 60, et contenant environ 20 % de l'isomère alkylène.

2. Siloxane à fonction benzalmalonate, susceptible d'être obtenu par :
a) dissolution de 13,28 g de {[4-(prop-2-ynyloxy)phényl]méthylène}-diéthylester dans 75 g de toluène et chauffage sous azote à environ 70°C,
b) addition goutte à goutte, après qu'un complexe de platine ait aussi été ajouté, de 44 g d'un hydrosiloxane présentant un degré de polymérisation de 65 et 6 (concentration maximum admise) groupe SiH (2,63 % de SiH) pour donner 10⁻⁴ moles de Pt par mole de SiH de l'hydrosiloxane,
c) chauffage au reflux du mélange et maintient à cette température jusqu'à ce que toutes les unités SiH aient disparues de l'analyse spectroscopique infrarouge,
d) retour du mélange à température ambiante, et
e) évaporation du toluène pour donner après lavage le polymère visqueux brun.
